# EUROPÄISCHE PATENTSCHRIFT

(11) **EP 0 666 317 B1**
(45) Veröffentlichungstag und Bekanntmachung des Hinweises auf die Patenterteilung: **07.12.2005**
(21) Anmeldenummer: 94114297.8
(22) Anmeldetag: 12.09.1994
(51) Int. Cl.: C12N 15/11, C07H 21/00, A61K 31/70

(54) **Antisense-Oligonucleotide gegen HSV-1 sowie deren Herstellung**
Antisense oligonucleotides against HSV-1 and their preparation
Oligonucléotides antisenses contre HSV-1 et leur préparation

(30) Priorität: 17.09.1993 DE 4331670
(43) Veröffentlichungstag der Anmeldung: 09.08.1995
(73) Patentinhaber: Sanofi-Aventis Deutschland GmbH, 65929 Frankfurt am Main (DE)
(72) Erfinder: Peyman, Anuschirwan, Dr., D-65779 Kelkheim (DE); Uhlmann, Eugen, Dr., D-61479 Glashütten (DE); Mag, Matthias, D-61440 Oberursel (DE); Kretzschmar, Gerhard, Dr., D-65760 Eschborn (DE); Helsberg, Matthias, Dr., D-65779 Kelkheim (DE); Winkler, Irvin, Dr., D-65835 Liederbach (DE)

(56) Entgegenhaltungen:
- EP-A- 0 653 439
- EP-A- 0 680 969
- WO-A-92/05284
- WO-A-95/01363
- CHEMICAL REVIEWS, Bd. 90, Nr. 4, 1.Juni 1990, Seiten 543-584, XP000141412 UHLMANN E ET AL: "ANTISENSE OLIGONUCLEOTIDES: A NEW THERAPEUTIC PRINCIPLE"
- NUCLEIC ACIDS RESEARCH, Bd. 19, Nr. 20, 1991, XP002014768 HOKE ET AL.: "Effects of phosphorothioate capping on antisense oligonucleotide stability, hybridization and antiviral efficacy versus herpes simplex virus infection"
- ANTIVIRAL RESEARCH, Bd. 13, 1990, Seiten 151-164, XP002014769 DRAPER ET AL.: "Complementary oligonucleotide sequence inhibits both Vmw65 gene expression and replication of herpes simplex virus"
- NUCLEOSIDES & NUCLEOTIDES, Bd. 14, Nr. 3-5, 7. - 11.September 1994, Seiten 1077-1081, XP000604879 PEYMAN ET AL.: "Enhanced cellular uptake of G-rich oligonucleotides"
- BIOL. CHEM. HOPPE-SEYLER, Bd. 376, 1995, Seiten 195-198, XP000604901 PEYMAN ET AL.: "Inhibition of viral growth by antisense oligonucleotides directed against the IE110 and the UL30 mRNA of Herpes simplex virus type-1"

### SEQUENZPROTOKOLL

(1) ALLGEMEINE INFORMATION:
   (i) ANMELDER:
      (A) NAME: HOECHST AKITENGESELLSCHAFT
      (B) STRASSE: -
      (C) ORT: Frankfurt am Main
      (D) BUNDESLAND: -
      (E) LAND: Deutschland
      (F) POSTLEITZAHL: 65926
      (G) TELEPHON: (069) 305-6031
      (H) TELEFAX: (069) 35 71 75
      (I) TELEX: 41234-700 hod
   (ii) ANMELDETITEL: Neue Antisense-Oligonucleotide gegen HSV-1 sowie deren Herstellung
   (iii) ANZAHL DER SEQUENZEN: 42
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 1:
   GCGGGGCTCC ATGGGGGTCG 20
(2) INFORMATION ZU SEQ ID NO: 2:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL, Mitte"
   (xi) SEQUENZBESCHREIBUNG: SEQ ID NO: 2:
   GCAGGAGGAT GCTGAGGAGG 20
(2) INFORMATION ZU SEQ ID NO: 3:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
   (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 3:
   GGGGCGGGGC TCCATGGGGG 20
(2) INFORMATION ZU SEQ ID NO: 4:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 4:
   GGCGGGGCTC CATGGGGGTC 20
(2) INFORMATION ZU SEQ ID NO: 5:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 5:
   GGGGCTCCAT GGGGGTCGTA 20
(2) INFORMATION ZU SEQ ID NO: 6:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL48, alpha-TIF, TI"
   (xi) SEQUENZBESCHREIBUNG: SEQ ID NO: 6:
   AAGAGGTCCA TTGGGTGGGG 20
(2) INFORMATION ZU SEQ ID NO: 7:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL52, Mitte"
   (xi) SEQUENZBESCHREIBUNG: SEQ ID NO: 7:
   GGCCCTGCTG TTCCGTGGCG 20
(2) INFORMATION ZU SEQ ID NO: 8:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 8:
   GGGCGGGGCT CCATGGGGGT 20
(2) INFORMATION ZU SEQ ID NO: 9:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 9:
   CCGGGGCGGG GCTCCATGGG 20
(2) INFORMATION ZU SEQ ID NO: 10:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 10:
   CGGGGCTCCA TGGGGGTCGT 20
(2) INFORMATION ZU SEQ ID NO: 11:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 11:
   GGGCTCCATG GGGGTCGTAT 20
(2) INFORMATION ZU SEQ ID NO: 12:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 12:
   GCTCCATGGG GGTCGTATGC 20
(2) INFORMATION ZU SEQ ID NO: 13:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL., TI"
   (xi) SEQUENZBESCHREIBUNG: SEQ ID NO: 13:
   CCGGAAAACA TCGCGGTTGT 20
(2) INFORMATION ZU SEQ ID NO: 14:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL., Mitte"
   (xi) SEQUENZBESCHREIBUNG: SEQ ID NO: 14:
   CCGGGGGCGC TTGGCCGGGG 20
(2) INFORMATION ZU SEQ ID NO: 15:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL., Mitte"
   (xi) SEQUENZBESCHREIBUNG: SEQ ID NO: 15:
   CAGCAGCTTG CGGGGCTTGG 20
(2) INFORMATION ZU SEQ ID NO: 16:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL., Mitte"
   (xi) SEQUENZBESCHREIBUNG: SEQ ID NO: 16:
   CCCCCAACAG GTGGGAGAAG 20
(2) INFORMATION ZU SEQ ID NO: 17:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL., Mitte"
   (xi) SEQUENZBESCHREIBUNG: SEQ ID NO: 17:
   GGGGGGTGCC ACACTTCGGG 20
(2) INFORMATION ZU SEQ ID NO: 18:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, 5'-untranslated"
   (xi) SEQUENZBESCHREIBUNG: SEQ ID NO: 18:
   CCCACCCGAA CCCCTAAAGA 20
(2) INFORMATION ZU SEQ ID NO: 19:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL48, alpha-TIF, TI"
   (xi) SEQUENZBESCHREIBUNG: SEQ ID NO: 19:
   GTCCGCGTTC ATGTCGGCAA 20
(2) INFORMATION ZU SEQ ID NO: 20:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL48, alpha-TIF, Mitte"
   (xi) SEQUENZBESCHREIBUNG: SEQ ID NO: 20:
   AACAGAGGCA GTCAAACAGG 20
(2) INFORMATION ZU SEQ ID NO: 21:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL48, 5'-untranslated"
   (xi) SEQUENZBESCHREIBUNG: SEQ ID NO: 21:
   ATACGGGAAA GACGATATCG 20
(2) INFORMATION ZU SEQ ID NO: 22:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL52, TI"
   (xi) SEQUENZBESCHREIBUNG: SEQ ID NO: 22:
   GTCTTCCTGC CCCATTGCGT 20
(2) INFORMATION ZU SEQ ID NO: 23:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL52, 5'-untranslated"
   (xi) SEQUENZBESCHREIBUNG: SEQ ID NO: 23:
   TGCGTCCGCG CGCCCAAGGG 20
(2) INFORMATION ZU SEQ ID NO: 24:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 24:
   GCGGGGCTCC ATGGGGGTCG 20
(2) INFORMATION ZU SEQ ID NO: 25:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 25:
   GCGGGGCTCC ATGGGGGTCG 20
(2) INFORMATION ZU SEQ ID NO: 26:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 26:
   GCGGGGCTCC ATGGGGGTCG 20
(2) INFORMATION ZU SEQ ID NO: 27:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL., Mitte"
   (xi) SEQUENZBESCHREIBUNG: SEQ ID NO: 27:
   GCAGGAGGAT GCTGAGGAGG 20
(2) INFORMATION ZU SEQ ID NO: 28:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL., Mitte"
   (xi) SEQUENZBESCHREIBUNG: SEQ ID NO: 28:
   GCAGGAGGAT GCTGAGGAGG 20
(2) INFORMATION ZU SEQ ID NO: 29:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL., Mitte"
   (xi) SEQUENZBESCHREIBUNG: SEQ ID NO: 29:
   GCAGGAGGAT GCTGAGGAGG 20
(2) INFORMATION ZU SEQ ID NO: 30:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL., Mitte"
   (xi) SEQUENZBESCHREIBUNG: SEQ ID NO: 30:
   GCAGGAGGAT GCTGAGGAGG 20
(2) INFORMATION ZU SEQ ID NO: 31:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "UL30, DNA-POL., Mitte"
   (xi) SEQUENZBESCHREIBUNG: SEQ ID NO: 31:
   GCAGGAGGAT GCTGAGGAGG 20
(2) INFORMATION ZU SEQ ID NO: 32:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 32:
   GCGGGGCTCC ATGGGGGTCG 20
(2) INFORMATION ZU SEQ ID NO: 33:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 33:
   GCGGGGCTCC ATGGGGGTCG 20
(2) INFORMATION ZU SEQ ID NO: 34:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 34:
   GCGGGGCTCC ATGGGGGTCG 20
(2) INFORMATION ZU SEQ ID NO: 35:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 35:
   GCGGGGCTCC ATGGGGGTCG 20
(2) INFORMATION ZU SEQ ID NO: 36:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 36:
   GGCGGGGCTC CATGGGGGTC 20
(2) INFORMATION ZU SEQ ID NO: 37:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 37:
   GGCGGGGCTC CATGGGGGTC 20
(2) INFORMATION ZU SEQ ID NO: 38:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..20
      (D) SONSTIGE ANGABEN: /note= "IE110, TI"
   (xi) SEQUENZBESCHREIBUNG: SEQ ID NO: 38:
   GGCGGGGCTC CATGGGGGTC 20
(2) INFORMATION ZU SEQ ID NO: 39:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: DNS (hypothetisch)
   (iii) HYPOTHETISCH: JA
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..21
   (xi) SEQUENZBESCHREIBUNG: SEQ ID NO: 39:
   CCAGGGTACA GGTGGCCGGC C 21
(2) INFORMATION ZU SEQ ID NO: 40:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: DNS (hypothetisch)
   (iii) HYPOTHETISCH: JA
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..21
   (xi) SEQUENZBESCHREIBUNG: SEQ ID NO: 40:
   CCAGGGTACA GGTGGCCGGC C 21
(2) INFORMATION ZU SEQ ID NO: 41:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: DNS (hypothetisch)
   (iii) HYPOTHETISCH: JA
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..21
   (xi) SEQUENZBESCHREIBUNG: SEQ ID NO: 41:
   CCAGGGTACA GGTGGCCGGC C 21
(2) INFORMATION ZU SEQ ID NO: 42:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: DNS (hypothetisch)
   (iii) HYPOTHETISCH: JA
   (iii) ANTISENSE: JA
   (vi) URSPRÜNLICHE HERKUNFT:
      (A) ORGANISMUS: HSV-1
   (ix) MERKMALE:
      (A) NAME/SCHLÜSSEL: exon
      (B) LAGE: 1..21
   (xi) SEQUENZBESCHREIBUNG: SEQ ID NO: 42:
   CCAGGGTACA GGTGGCCGGC C 21

## Beschreibung

Humane Herpesviren sind durch eine Reihe von Gemeinsamkeiten charakterisiert, z. B. gemeinsame strukturelle Morphologie, bestimmte Aspekte der Replikation und der Fähigkeit, lebenslange Infektionen zu verursachen. Dies gilt insbesondere für Herpes Simplex Viren vom Typ 1 und Typ 2 (HSV-1 und HSV-2). HSV verursacht ein breites Spektrum an Krankheiten, die von relativ milden Läsionen der Haut, der Mucus-Membran und des Corneal Epithels bis hin zu schweren und oft tödlich verlaufenden Fällen von Enzephalitis reichen. Periodische Reaktivierungen des latenten Virus während der Lebenszeit des Wirts resultieren in wiederkehrenden Hautläsionen, oft begleitet von signifikanter Morbidität.

Für die Antivirale Therapie von HSV gibt es verschiedene Ansätze. So inhibieren beispielsweise Antisense-Oligonucleotide selektiv die virale Genexpression und haben daher das Potential, Zellen auf molekularer Ebene vor Latenz oder vor Reaktivierung zu schützen.

Antisense-Oligonucleotide sind komplementär zu einer spezifischen messenger RNA-Sequenz, binden spezifisch an diese und inhibieren auf diese Weise spezifisch die Genexpression [E. Uhlmann und A. Peyman, Chem. Rev. 90 (1990) 543].

Neben den bekannten Anforderungen für Antisense-Oligonucleotide wie beispielsweise Nucleasestabilität, Zellgängigkeit etc. ist die Wahl der Target-Sequenz für die Wirkung der Antisense-Oligonucleotide von großer Bedeutung. Obwohl meist viele Regionen der RNA für eine Hybridisierung in Frage kommen, gibt es Bereiche, die eine besonders starke Inhibierung der Genexpression bewirken. Die Anforderungen zu Nucleasestabilität, Zellgängigkeit etc. lassen sich durch chemische Modifikationen erzielen, beispielsweise durch Modifikation der Phosphatbrücke, Variation des Zuckerbausteins etc. Die Wahl der Target-Sequenz wird lediglich bestimmt durch die Reihenfolge der Basen während andere Strukturelemente, wie etwa das Zucker-Phosphat-Rückgrat, modifiziert werden können, vorausgesetzt, die Fähigkeit zur Hybridisierung wird durch die Modifikation nicht eingeschränkt (beispielsweise Phosphorothioate anstelle von Phosphorsäurediestern). Auch modifizierte Basen können unter der Vorraussetzung eingesetzt werden, daß sich durch die Modifikation die Spezifität der Watson-Crick-Basenpaarung nicht ändert, (beispielsweise 5-Methylcytosin für Cytosin).

Über die Verwendung von Antisense-Oligonucleotiden, die komplementär zu bestimmten Regionen des HSV-1 Genoms sind, zur Bekämpfung des Virus wurde berichtet.

Zusammenfaßend läßt sich sagen, daß "All"-Phosphorothioate, d.h. Oligonucleotide mit ausschließlich Phosphorothioatbrücken anstelle von Phosphorsäurediesterbrücken, Inhibitoren von HSV-1 in Zellkultur sind (siehe hierzu beispielsweise J.F. Milligan et al., J. Med. Chem. 36 (1993) 1923). Ein 21-meres Phosphorothioat Antisense-Oligonucleotid gegen die UL13 mRNA inhibiert das Virus über 90 % bei 4 µM (G. D. Hoke et al., Nucleic Acids Res 19 (1991) 5743). Die gleiche Sequenz ist nicht aktiv, wenn sie als nichtmodifiziertes Oligonucleotid oder als Oligonucleotid eingesetzt wird, welches am 5'- oder 3'-Ende jeweils drei Phosphorothioat-Brücken trägt. Gleichzeitig zeigen "All"-Phosphorothioat-Oligonucleotide erhebliche unspezifische Effekte, die auf andere Mechanismen als auf den Antisense-Mechanismus zurückzuführen sind. So zeigt beispielsweise S-(dC)₂₈ schon bei 1 µM eine Inhibierung von HSV-1 von 90 % (W. Gao et al., J. Biol. Chem. 265 (1990) 20172).

Eine weitere Verbindungsklasse die gegen HSV-1 getestet wurde sind Methylphosphonat-Oligonucleotide. Eine Serie von vier 12-meren Antisense-Oligonucleotiden, die gegen überlappende Sequenzen des Exon-Intron Bereichs der 5'-Splice acceptor site des IE 4-Gens von HSV-1 gerichtet waren, wurden individuell bei 100 *µ*M getestet und erzielten lediglich breit streuende Ergebnisse der Virus-Inhibierung von 9-98 %. Ein 12-meres Antisense-Oligonucleotid gegen den Translationsstart von IE110 zeigte ebenfalls nur eine minimale bzw. keine Inhibierung von HSV-1 (M. Kulka et al., Proc. Natl. Acad. Sci. U. S. A. 86 (1989) 6868, L. Aurelian et al. WO 92/05284 [PCT/US91/06646]).

Überraschenderweise haben wir gefunden, daß es keinesfalls ausreichend ist, ein Antisense-Oligonucleotid gegen einen, selbst eingeschränkten, Sequenzabschnitt eines Target-Gens zu richten, sondern, daß vielmehr minimale Verschiebungen entlang der Target-Sequenz zu großen Schwankungen in der Aktivität führen können. Beispielsweise ist es nicht ausreichend, ein Antisense-Oligonucleotid complementär zum Translationsstart des IE110-Gens von HSV-1 zu verwenden, sondern vielmehr erweisen sich aus der großen Zahl denkbarer Sequenzen nur einige wenige als aktiv gegen HSV-1, wie in Tabelle 1 verdeutlicht wird. Andere wiederum zeigen unter den angewandten Testbedingungen (Tests nur mit Konzentrationen kleiner 80 µM an Oligonucleotid) keine oder nur minimale Aktivität. Vergleichbare Ergebnisse konnten auch für andere Target-Gene gefunden werden (Tabelle 2-4). Überraschenderweise konnten wir Antisense-Oligonucleotide auffinden, die gegen HSV-1 gute Aktivität zeigen obwohl sie nur solche minimalen chemische Modifikationen aufweisen (am 5'- oder 3'-Ende jeweils zwei Phosphorothioat-Brücken), die in den bisher beschriebenen "besten" Sequenzen gegen HSV-1 keine Aktivität zeigten (G. D. Hoke et al., Nucleic Acids Res 19 (1991) 5743).

Gegenstand der vorliegenden Erfindung sind daher Antisense-Oligonucleotide mit der Sequenz: oder sowie deren Mischungen.

Bevorzugt sind die Sequenzen: oder

Besonders bevorzugt ist die Sequenz:

Zum Gegenstand der Erfindung gehören ebenso um an den jeweiligen Enden unabhängig voneinander bis zu zwei Nukleotide, vorzugsweise bis zu einem Nukleotid verkürzte oder verlängerte erfindungsgemäße Oligonukleotide. Zusätzlich gehören zum Gegenstand der Erfindung deren 90 %ige, vor allem 95 %ige Homologe sowie modifizierte Antisense-Oligonukleotide.

Unter modifizierten Antisense-Oligonucleotiden sind dabei chemische Modifikationen zu verstehen, die die Eigenschaften von Antisense Oligonucleotiden, wie beispielsweise Nuclease-Stabilität und Zellgängigkeit, verbessern, und die dem Fachmann bekannt sind (beispielsweise: E. Uhlmann und A. Peyman, Chem. Rev. 90 (19901 543; P. D. Cook, Anti-Cancer Drug Design 6 (1991) 585); J. Goodchild, Bioconjugate Chem. 1 (1990) 165; S. L. Beaucage und R. P. lyer, Tetrahedron 49 (1993) 6123; F. Eckstein, Ed., "Oligonucleotides and Analogues-A Practical Approach", IRL Press 1991.)

Solche Modifikationen sind beispielsweise:
a) Modifikationen der Phosphatbrücke, beispielhaft seien genannt Phosphorothioate, Phosphorodithioate, Methylphosphonate, Phosphoramidate, Boranophosphate, Phosphatmethylester, Phosphatethylester, Phenylphosphonate. Bevorzugte Modifikationen der Phosphatbrücke sind Phosphorothioate und Methylphosphonate. Ausgenommen soll sein der Ersatz aller Phosphatbrücken der erfindungsgemäßen Oligonucleotide durch die genannten Modifikationen.
b) Ersatz der Phosphatbrücke, beispielhaft seien genannt der Ersatz durch Formacetal, 3'-Thioformacetal, Methylhydroxylamin, Oxim, Methylendimethylhydrazo, Dimethylensulfon, Silylgruppen. Bevorzugt ist der Ersatz durch Formacetale und 3'-Thioformacetale.
c) Modifikationen des Zuckers, beispielhaft seien genannt α-anomere Zucker, 2'-O-Methylribose, 2'-O-Butylribose, 2'-O-Allylribose, 2'-Fluoro-2'-desoxyribose, 2'-Amino-2'-desoxyribose, α-Arabinofuranose, Carbocyclische Zuckeranaloge. Bevorzugte Modifikation ist die durch 2'-O-Methylribose.
d) Modifikationen der Basen welche die Spezifität der Watson-Crick Basenpaarung nicht verringern, beispielhaft seien genannt 5-Propin-2'-desoxyuridin, 5-Propin-2'-desoxycytidin, 5-Fluoro-2'-desoxycytidin, 5-Fluor-2'-desoxyuridin, 5-Hydroxymethyl-2'-desoxyuridin, 5-Methyl-2'-desoxycytidin, 5-Brom-2'-desoxycytidin. Bevorzugte Modifikationen sind 5-Propin-2'-desoxyuridin und 5-Propin-2'-desoxycytidin.
e) Ersatz des Zuckerphosphat-Rückgrats, beispielsweise durch "Morpholinonucleosid"-Oligomere [E. P. Stirchak et al., Nucleic Acids Res. 17 (1989) 6129] oder durch "Peptide Nucleic Acids" [z.B. Hanvey et al., Science 258 (1992) 1481].
f) 5'- und 3'-Phosphate, sowie 5'- und 3'-Thiophosphate.
g) Konjugate, beispielsweise am 3'- oder am 5'-Ende (siehe auch EP-A2-0 552 766 zur 3'-Derivatisierung); beispielhaft seien Konjugate genannt mit DMAB-3', Poly-Lysin, mit Interkalatoren wie Pyren, Acridin, Phenazin, Phenanthridin, mit fluoreszierenden Verbindungen wie Fluorescein, mit Cross-Linkern wie Psoralen, Azidoproflavin, mit lipophilen Molekülen wie C₁₂-C₂₀-Alkyl, mit Lipiden wie 1,2-di-hexadecyl-rac-glycerin, mit Steroiden wie Cholesterin oder Testosteron, mit Vitaminen wie Vitamin E, mit Poly-bzw. Oligo-ethylengylcol, mit (C₁-C₁₈)-Alkyl-Phosphatdiestern, mit -O-CH₂-CH(OH)-O-(C₁₂-C₁₈)-Alkyl, insbesondere Konjugate mit Pyrenderivaten, wie beispielsweise die erfindungsgemäße Verbindung AO26.
h) 3'-3'- und 5'-5'-Inversionen [z.B. M. Koga et al., J. Org. Chem. 56 (1991) 3757].

Besonders bevorzugt sind die folgenden Modifikationen:
a) Ersatz der Phosphatbrücken durch Phosphorothioate, Phosphorodithioate oder Methylphosphonate. Ausgenommen soll sein der Ersatz aller Phosphatbrücken der erfindungsgemäßen Oligonucleotide durch die genannten Modifikationen.
b) Konjugate mit lipophilen Molekülen wie C₁₂-C₂₀-Alkyl, mit Steroiden wie Cholesterin oder Testosteron, mit Poly- oder Oligoethylenglykol, mit Vitamin E, mit Interkalatoren wie Pyren, mit (C₁₄-C₁₈)-Alkyl-Phosphatdiestern, mit - O-CH₂-CH(OH)-O-(C₁₂-C₁₆)-Alkyl.

Vor allem sind folgende Modifikationen bevorzugt:
a) Der Ersatz von ein, zwei oder drei Phosphatbrücken am 5'- und/oder 3'-Ende durch Phosphorothioate, insbesondere der Ersatz von zwei Phosphatbrücken am 5'- und 3'-Ende durch Phosphorothioate oder Phosphorodithioate.
b) Der Ersatz von ein, zwei oder drei Phosphatbrücken am 5'- und/oder 3'-Ende durch Phosphorothioate und zusätzlich der Ersatz von 1-5 Phosphatbrücken durch Phosphorothioate in der Mitte.
c) Konjugate mit Polyethylenglycol, Hexaethylenglycol, Cholesterin, Testosteron, Pyren, Batyl, Vitamin E, mit (C₁₄-C₁₈)-Alkyl-Phosphatdiestern, mit -O-CH₂-CH(OH)-O-(C₁₂-C₁₆)-Alkyl, insbesondere in Verbindung mit dem Ersatz von ein, zwei oder drei Phosphatbrücken am 5'- und/oder 3'-Ende durch Phosphorothioate.

Gegenstand der Erfindung sind weiterhin Verfahren zur Herstellung der erfindungsgemäßen Verbindungen nach dem Fachmann bekannten Verfahren, insbesondere die chemische Synthese, die Verwendung der erfindungsgemäßen Verbindungen zur Herstellung eines Arzneimittels gegen HSV-1, ein Verfahren zur Herstellung eines Arzneimittels gegen HSV-1, dadurch gekennzeichnet, daß man eine oder mehrere der erfindungsgemäßen Verbindung mit einem physiologisch annehmbaren Träger sowie gegebenenfalls geeigneten Zusatz- und/oder Hilfsstoffen vermischt, sowie die Verwendung von der erfindungsgemäßen Verbindungen zur Herstellung eines Arzneimittels gegen HSV-1.

Eine bevorzugte Form der Verabreichung ist die Injektion. Hierzu werden die Antisense-Oligonucleotide in einer flüssigen Lösung, vorzugsweise in einem physiologisch annehmbaren Puffer, wie z.B. Hank's Lösung oder Ringer's Lösung, formuliert. Die Antisense-Oligonucleotide können aber auch in fester Form formuliert werden und vor dem Gebrauch gelöst bzw. suspendiert werden. Die für die systemische Verabreichung bevorzugten Dosierungen betragen ca. 0,01 mg/kg bis ca. 50 mg/kg Körpergewicht und Tag.

Die folgenden Beispiele sollen die Erfindung näher erläutern.

### Beispiel 1

### Oligonucleotidsynthese

Unmodifizierte Oligonucleotide wurden auf einem automatischen DNA Synthesizer (Applied Biosystems Model 380B oder 394) unter Anwendung der Standard Phosphoramidit-Chemie und Oxidation mit Jod synthetisiert. Zur Einführung von Phosphorthioat-Brücken in gemischten Phosphorothioaten und Phosphodiester Oligonucleotiden wurde anstelle von Jod mit TETD (Tetraethylthiuramdisulfid) oxidiert (Applied Biosystems User Bulletin 65). Nach Abspaltung vom festen Träger (CPG oder Tentagel) und Entfernung der Schutzgruppen mit konz. NH₃ bei 55°C während 18h, wurden die Oligonucleotide zunächst durch Butanol-Fällung (Sawadogo, Van Dyke, Nucl. Acids Res. 19 (1991) 674) gereinigt. Anschließend wurde das Natriumsalz erhalten durch Ausfällung aus einer 0.5 M NaCl Lösung mit 2.5 Volumenteilen Ethanol.

Die Einführung des [4-(1-pyrenyl)butanyl]phosphodiesters am 5'-Ende erfolgt wie in J. S. Mann et al., Bioconj. Chem. 3 (1992) 554 beschrieben.

Die Analyse der Oligonucleotide erfolgte durch
a) Analytische Gelelektrophorese in 20 % Acrylamid, 8M Harnstoff, 45mM Tris-borat Puffer, pH 7,0 und/oder
b) HPLC-Analyse: Waters GenPak FAX, Gradient CH₃CN (400 ml), H₂O (1,6l), NaH₂PO₄ (3,1 g), NaCl (11,7 g), pH6,8 (0,1 M an NaCl) nach CH₃CN (400 ml), H₂O (1,6 l), NaH₂PO₄ (3,1 g), NaCl (175,3 g), pH6,8 (1,5 M an NaCl)
   und/oder
c) Kapillargelelektrophorese Beckmann Kapillare eCAP™, U100P Gel Column, 65 cm length, 100 mm I.D., window 15 cm from one end, Puffer 140 µM Tris, 360µM Borsäure, 7M Harnstoff.
   und/oder
d) Elektrospray Massenspektroskopie.

Die Analytise der Oligonucleotide ergab, daß diese jeweils in einer Reinheit von größer 90 % vorlagen.

Folgende Oligonucleotide wurden synthetisiert:

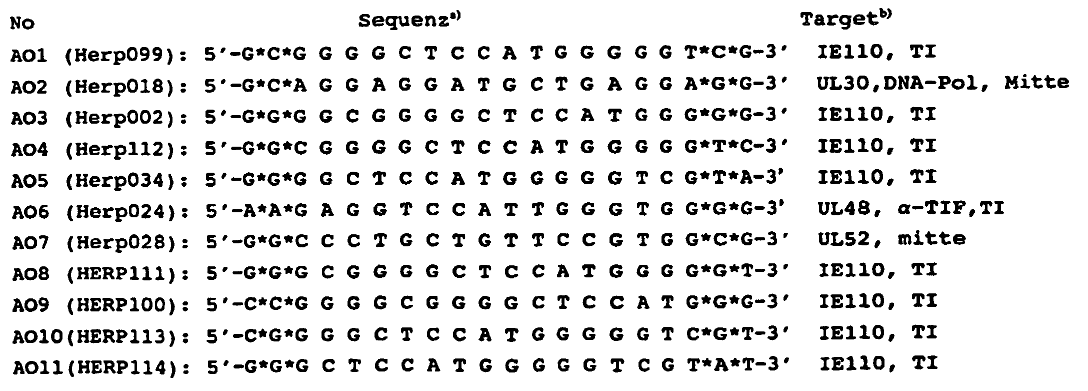

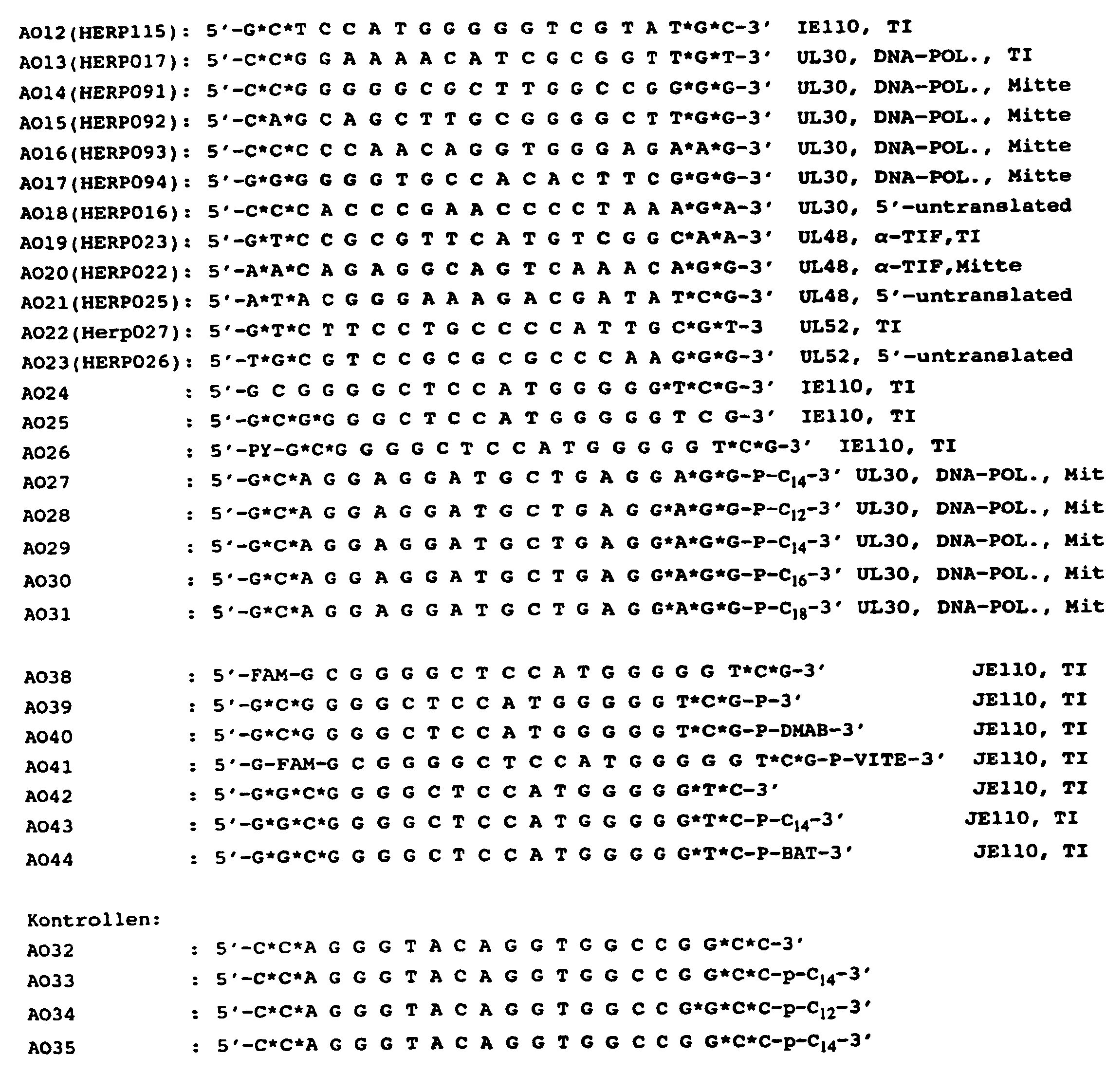

a) Die durch eine Phosphorothioatbrücke ersetzte Phosphodiesterbindungen wurden in der Sequenz mit * markiert; 5'-PY bedeutet einen 5'-[4-(1-pyrenyl)butanyl]phosphodiester;
b) Targetsequenz auf dem Genom von HSV-1: TI: Translation Initiation Site des Target-Gens, Mitte: Mitte des Target-Gens.
c) Die 3'-derivatisierten Oligonucleotide wurden wie in EP 0552766 A2 beschrieben synthetisiert. Dabei bedeuten p-C₁₂-3' einen 3'-n-C₁₂H₂₅-Phosphorsäureester, p-C₁₄ einen 3'-n-C₁₄H₂₉-Phosphorsäureester, p-C₁₆ einen 3'-n-C₁₆H₃₃-Phosphorsäureester, p-C₁₈ einen 3'-n-C₁₈H₃₇-Phosphorsäureester; P-3' steht für ein 3'Phosphat; P-VITE steht für einen 3'-Vitamin E-Phosphorsäureester, P-BAT-3'- steht für einen 3'-Batyl-Phosphorsäureester; DMAB-3' steht für 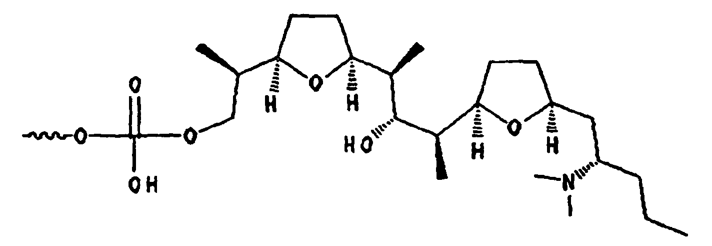 und wurde über einen derivatisierten Träger (DMTr: Dimethoxytrityl, CPG: Controlled Pore Glass) 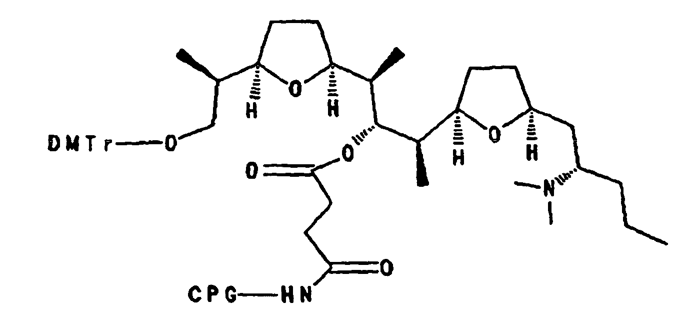 mit der Standard-Phosphoramidit-Chemie eingeführt. 5'-FAM steht für 3-Hydroxy-2-(N-thioharnstoff-fluorescein-4-aminobutyl)-propyl-1-O-phosphorsäureester, der über das entsprechende Phosphoramidit wie in F. Schubert et al., Nucl. Acids Res. 18 (1990) 3427 eingeführt wird.

### Beispiel 2

### Untersuchung der antiviralen Aktivität von Prüfsubstanzen gegen Herpesviren in vitro

Die antivirale Aktivität der Prüfsubstanzen gegen verschiedene humanpathogene Herpesviren wird im Zellkulturtestsystem untersucht.

Für den Versuch werden Affennierenzellen (Vero, 2x10⁵/ml) in serumhaltigem Dulbecco's MEM (5 % Fötales Kälberserum FCS) in 96-Napf-Mikrotiterplatten ausgesät und 24 h bei 37°C und 5 % CO₂ inkubiert. Das serumhaltige Medium wird dann abgesaugt und die Zellen werden zweimal mit serumfreiem Dulbecco's MEM (-FCS) überspült.

Die Testsubstanzen werden in H₂O auf eine Konzentration von 600 µM vorverdünnt und bei -18°C aufbewahrt. Für den Test erfolgen weitere Verdünnungsschritte in Dulbecco's Minimal Essential Medium (MEM). Je 100 µl der einzelnen Prüfsubstanzverdünnungen werden zusammen mit 100 µl serumfreiem Dulbecco's MEM (-FCS) zu den gespülten Zellen gegeben.

Nach 3 h Inkubation bei 37°C und 5 % CO₂ werden die Zellen mit Herpes simplex Virus Typ 1 (ATCC VR733, HSV-1 F-strain) infiziert in Konzentrationen, bei denen der Zellrasen innerhalb von 3 Tagen vollkommen zerstört wird. Bei HSV-1 beträgt die Infektionsstärke 500 Plaque-bildende Einheiten (PFU) pro Napf, bei HSV-2 350 PFU/Napf. Die Versuchsansätze enthalten dann Prüfsubstanz in Konzentrationen von 80 *µ*M bis 0,04 *µ*M in MEM, ergänzt durch 100 U/ml Penicillin G und 100 mg/l Streptomycin. Alle Versuche werden als Doppelbestimmung durchgeführt mit Ausnahme der Kontrollen, die achtfach je Platte durchgeführt werden.

Die Versuchsansätze werden 17 h bei 37°C und 5 % CO₂ inkubiert. Die Cytotoxizität der Prüfsubstanzen wird nach 20 h Gesamtinkubationszeit durch mikroskopische Begutachtung der Zellkulturen bestimmt. Als Dosis tolerata maxima (DTM) wird die höchste Präparatkonzentration bezeichnet, die unter den genannten Versuchsbedingungen noch keine mikroskopisch erkennbaren Zellschädigungen hervorruft.

Es erfolgt daraufhin die Zugabe von FCS auf eine Endkonzentration von 4 % mit weiterer Inkubation für 55 h bei 37°C und 5 % CO₂. Die unbehandelten Infektionskontrollen zeigen dann einen kompletten cytopathischen Effekt (CPE). Nach mikroskopischer Begutachtung der Zellkulturen werden diese dann mit Neutralrot entsprechend dem Vitalfärbungsverfahren nach Finter (1966) angefärbt. Die antivirale Aktivität einer Testsubstanz wird definiert als minimale Hemmkonzentration (MIC), die benötigt wird, um 30-60 % der Zellen vor dem virusbedingten cytopathogenen Effekt zu schützen.

Die Ergebnisse der Versuche sind in den Tabellen 1-6 dargestellt.

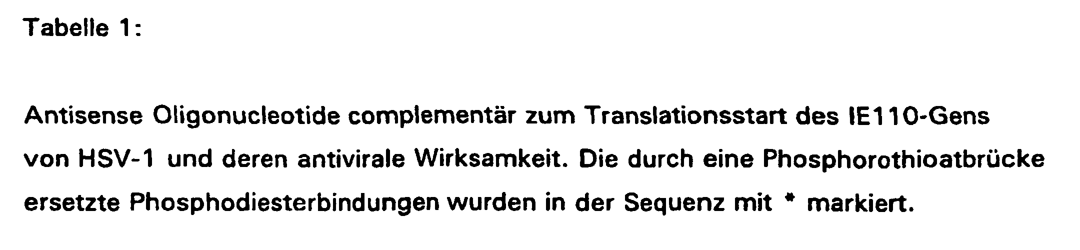

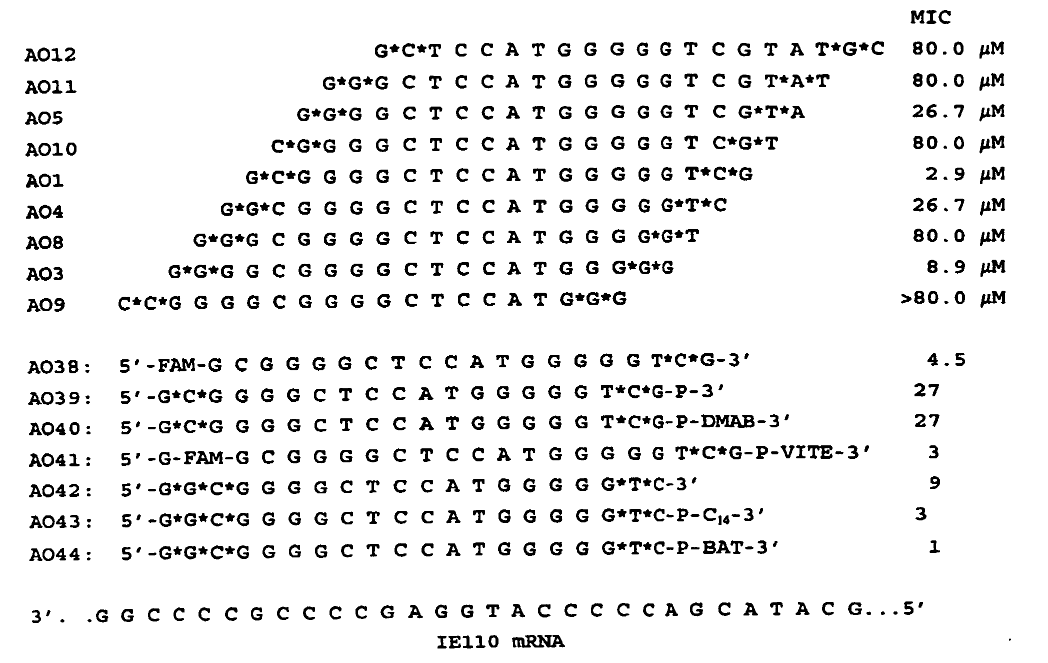

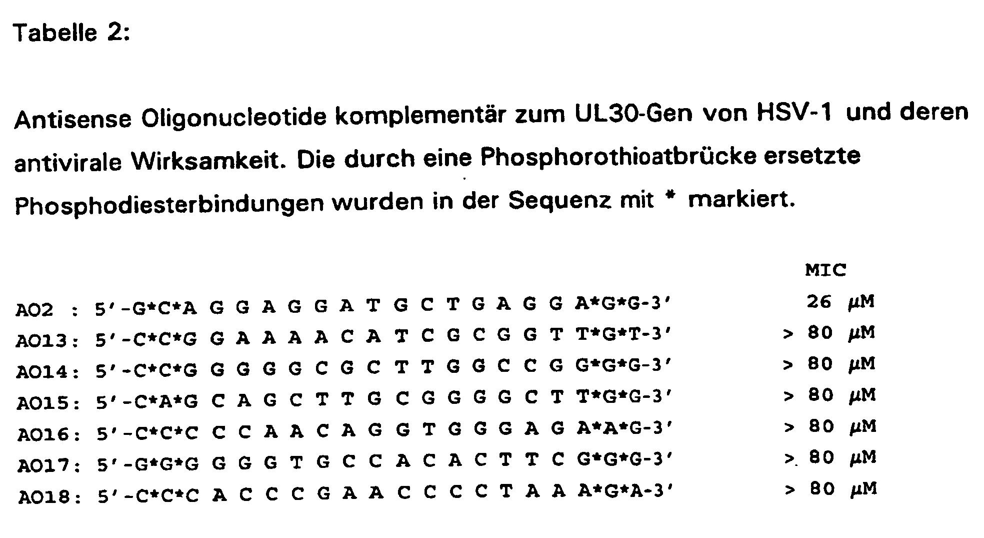

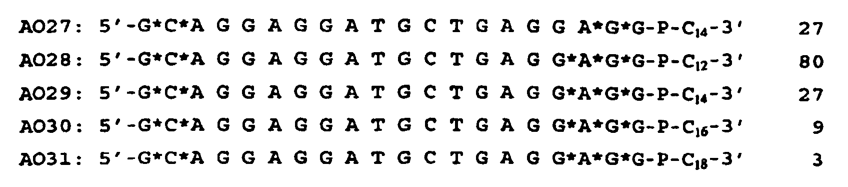

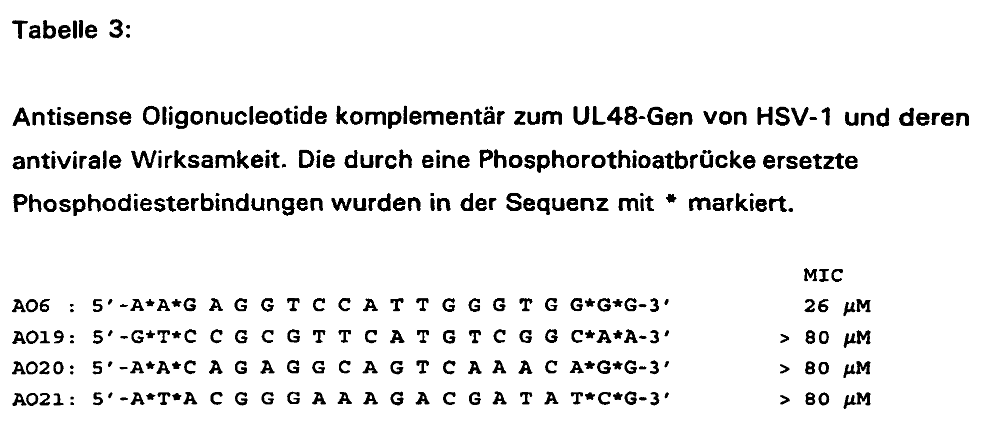

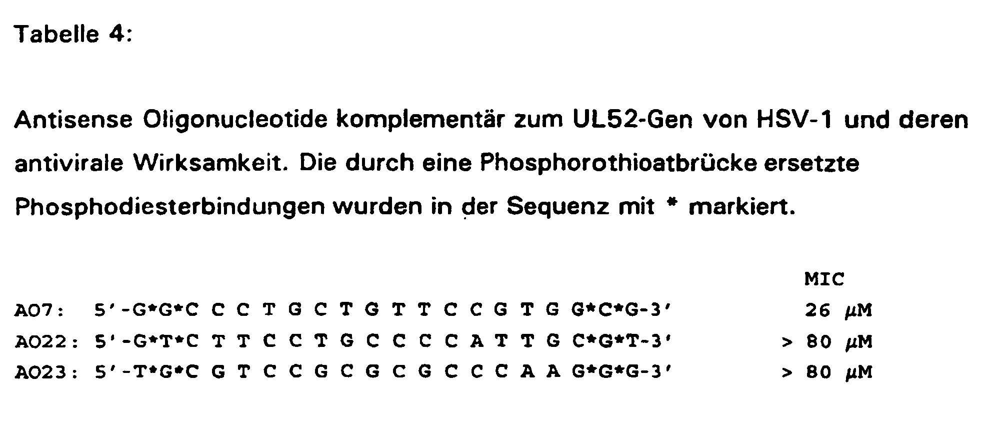

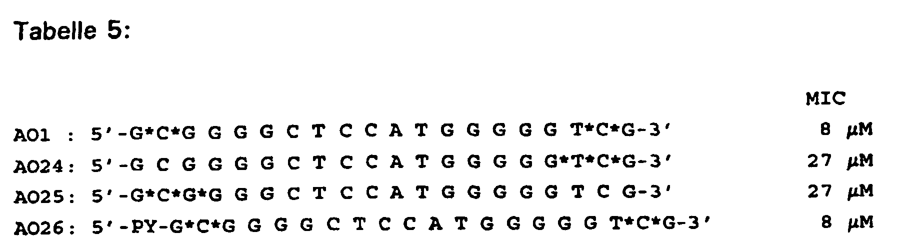

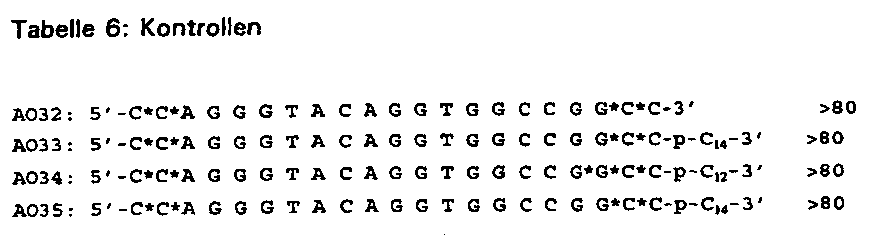

### Beispiel 3

### Untersuchung der antiviralen Aktivität von Prüfsubstanzen gegen Herpesviren in vivo

NMRI-Mäuse (NMRI: Naval Medical Research Insitute), spezifisch pathogenfrei, im Gewicht von etwa 15 g wurden intraperitoneal mit Herpes simplex Typ 1 infiziert und anschließend mit der unten beschriebenen Verbindungen intraperitoneal, subcutan oder per os therapiert. Die Dosierung der erfindungsgemäßen Verbindungen lag zwischen 1 und 100 *µ*g. Die Behandlung erfolgte zweimal täglich über 2,5 Tage, beginnend nach der Infektion. Der Behandlungserfolg wurde anhand des Krankheitsverlaufes und der Überlebensrate gegenüber den unbehandelten Infektionskontrollen bestimmt. Letztere erhielten anstelle der zu prüfenden Verbindungen eine wasserlösliche Methylhydroxyethylcellulose (Viskosität 300 Pa in 2 % Lösung) ((R) Tylose MH 300 P) appliziert.

Folgenes Oligonucleotid wurde getestet:

Eine präliminäre Auswertung ergab, daß das geprüfte Oligonucleotid im getesteten Konzentrationsbereich gegenüber den Kontrollen wirksam war.

## Patentansprüche

1. Antisense-Oligonucleotide mit der Sequenz oder wobei das Symbol * bedeutet, dass die Phosphodiesterbindung durch eine Phosphothioatbrücke ersetzt wurde.

2. Antisense-Oligonucleotide nach Anspruch 1, **dadurch gekennzeichnet, daß** die Oligonucleotide mit 3'-Phosphat, Fluorescein, lipophilen Molekülen, Steroiden, Polyethylenglycol, Oligoethylenglycol, Vitamin E, Interkalatoren, (C₁-C₁₈)-Alkyl-Phosphatdiestern, gegebenenfalls ein- oder mehrfach mit Cl-, Br- und/oder OH-substituiert, oder mit -O-CH₂-CH(OH)-O-(C₁₂-C₁₆)-Alkyl verknüpft sind.

## Claims

1. An antisense oligonucleotide having the sequence or where the symbol * means that the phosphodiester bond is replaced by a phosphothioate bridge.

2. An antisense oligonucleotide as claimed in claim 1, which oligonucleotide is linked to 3'-phosphate, fluorescein, lipophilic molecules, steroids, polyethylene glycol, oligoethylene glycol, vitamin E, intercalating agents, (C₁-C₁₈)-alkyl phosphate diesters, optionally substituted once or more than once by Cl, Br and/or OH, or to -O-CH₂-CH(OH)-O-(C₁₂-C₁₆) -alkyl.

## Revendications

1. Oligonucléotides antisens de séquences : ou dans lesquelles le symbole * signifie que la liaison phosphodiester est remplacée par un pont phosphothioate.

2. Oligonucléotides antisens selon la revendication 1, **caractérisés en ce que** les oligonucléotides sont combinés à du 3'-phosphate, de la fluorescéine, des molécules lipophiles, des stéroïdes, du polyéthylène-glycol, de l'oligoéthylène-glycol, de la vitamine E, des agents intercalants, des diesters de phosphate d'alkyle (en C₁ à C₁₈), éventuellement substitués une ou plusieurs fois par Cl, Br et/ou OH, ou à un radical -O-CH₂-CH (OH) -O-alkyle (en C₁₂ à C₁₆).
